# EUROPEAN PATENT APPLICATION

(11) **EP 4 108 648 A1**
(43) Date of publication of application: **28.12.2022**
(21) Application number: 21757471.4
(22) Date of filing: 18.02.2021
(51) Int. Cl.: C07B 59/00, C07C 45/63, C07C 269/06, C07C 271/22, C07D 261/20, C07D 333/62, C07J 1/00, C07C 49/697, C07D 209/48, C07D 215/18, C07C 67/307, C07C 69/712

(54) **METHOD FOR PRODUCING AROMATIC ASTATINE COMPOUND**

(30) Priority: 21.02.2020 JP 2020028536
(71) Applicant: National University Corporation Hokkaido University, Sapporo-shi, Hokkaido 060-0808 (JP)
(72) Inventor: MATSUNAGA, Shigeki, Sapporo-shi, Hokkaido 060-0808 (JP); YOSHINO, Tatsuhiko, Sapporo-shi, Hokkaido 060-0808 (JP); MATSUOKA, Keitaro, Sapporo-shi, Hokkaido 060-0808 (JP); OGAWA, Mikako, Sapporo-shi, Hokkaido 060-0808 (JP)
(74) Representative: Murgitroyd & Company
(86) International application number: PCT/JP2021/006133
(87) International publication number: WO 2021/167008

(57) **Abstract**

This method for producing an aromatic astatine compound comprises reacting an aromatic iodonium ylide with astatine to produce an aromatic astatine compound.

## Description

### [Technical Field]

The present invention relates to a method of producing an aromatic astatine compound, more particularly a method of producing an aromatic astatine compound by a reaction between an aromatic iodonium ylide and astatine.

### [Background Art]

[²¹¹At]astatine is a promising radioisotope for *α*-ray therapy. For the synthesis of ²¹¹At-labeled *α*-ray therapeutic agents, ²¹¹At labeling reactions for aromatic rings have been developed and studied. For example, aromatic electrophilic substitution reactions in which an electrophilic astatine species acts on an aryl stannane (an aromatic tin compound) have been developed. However, electrophilic astatine has problems in that it may cause unnecessary side reactions since it exists in multiple oxidation states, and that electrophilic astatine species present a risk in clinical applications due to their high volatility. Thus, labeling methods using nucleophilic astatine species, which is a single chemical species with low volatility and relatively high safety, have been studied.

Non-patent Literature 1 reports a method of producing an aromatic astatine-labeled compound by allowing a diaryliodonium salt as a labeling precursor to react with astatine. However, this method has a problem in that the applicable range of a method of producing the diaryliodonium salt serving as a labeling precursor is limited to simple aromatic rings, and that it is difficult to control the chemoselectivity as to which of the two aromatic rings of the diaryliodonium salt undergoes the reaction.

Non-patent Literature 2 reports a method of producing an aromatic astatine-labeled compound by allowing an aryl boronic ester as a labeling precursor to react with astatine in the presence of a copper catalyst. However, this production method requires the use of a transition metal reagent (catalyst), and thus has a problem in clinical applications from the safety standpoint.

Meanwhile, Non-patent Literature 3 discloses that aromatic compounds can be ¹⁸F-fluorinated through a reaction between iodonium ylide and ¹⁸F.

### [Citation List]

### [Non-Patent Literatures]

[Non-patent Literature 1] Francois Guerard, Yong-Sok Lee, Kwamena Baidoo, Jean-Francois Gestin, and Martin W. Brechbiel, Chem, Eur, J. 2016, 22, 12332-12339
[Non-patent Literature 2] Sean W. Reilly, Mehran Makvandi, Kuiying Xu, and Robert Mach, Org. Lett. 2018, 20, 1752-1755
[Non-patent Literature 3] Benjamin H. Rotstein, Nickeisha A. Stephenson, Neil Vasdev, and Steven H. Liang, Nature Communications 2014, 4365

### [Summary of Invention]

### [Technical Problem]

In consideration of clinical applications, it is believed useful to utilize "nucleophilic astatine that is a relatively safe and simple chemical species" as a raw material of an intended ²¹¹At labeling reaction. In addition, from the safety standpoint, it is believed important not to use a "transition metal" in the raw material of the reaction and not to use a "transition metal" as a catalyst. Further, the reaction is more preferably "capable of achieving ²¹¹At labeling in a chemoselective and regioselective manner". Such a ²¹¹At labeling reaction has not been known and is of interest both academically and commercially.

The present inventors have reported various aromatic iodonium ylide compounds and their production methods (Non-patent Literature 4: Keitaro Matsuoka, Narumi Komami, Masahiro Kojima, Tatsuhiko Yoshino, and Shigeki Matsunaga, Asian J. Org. Chem. 2019, 8, 1107-1110, and Non-patent Literature 5: Narumi Komami, Keitaro Matsuoka, Ayako Nakano, Masahiro Kojima, Tatsuhiko Yoshino, and Shigeki Matsunaga, Chem. Eur. J. 2019, 25, 1217-1220).

If a ²¹¹At labeling reaction can be performed using any of these aromatic iodonium ylide compounds, a clinically applicable ²¹¹At labeling reaction, in which nucleophilic astatine can be utilized and which has excellent safety and can simplify the resulting product, more preferably has excellent chemoselectivity and regioselectivity, is expected to be obtained.

Incidentally, fluorine atom and astatine both belong to the same halogen family.

Non-patent Literature 3 discloses a reaction of an aromatic iodonium ylide and fluorine; however, it does not offer any disclosure or teaching with regard to a reaction of an aromatic iodonium ylide and astatine.

Further, Non-Patent Literature 1 (particularly, see lines 21 to 25 in the right column of Introduction on page 12,332) discloses that the behavior of astatine is different from those of other halogens and is not well known.

### [Solution to Problem]

The present inventors intensively studied to discover that a ²¹¹At-labeled aromatic astatine compound is obtained by a ²¹¹At labeling reaction using an aromatic iodonium ylide compound, and that the ²¹¹At-labeled aromatic astatine compound is suitable for the use in α-radiolabeled therapeutic agents, thereby completing the present invention.

The present specification includes the following embodiments.
1. A method of producing an aromatic astatine compound, the method comprising allowing an aromatic iodonium ylide to react with astatine to produce the aromatic astatine compound.
2. The method according to 1, wherein the aromatic iodonium ylide is represented by the following Formula (1): 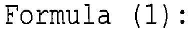 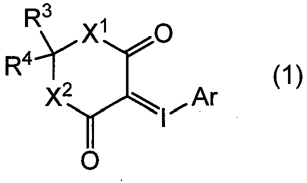 [wherein,
   Ar represents an aromatic group optionally having a substituent and optionally having a heteroatom;
   X¹ is selected from a group consisting of NR¹, O, and S;
   X² is selected from a group consisting of NR², O, and S;
   R¹ and R² are each independently selected from H, alkyls optionally having a substituent and optionally interrupted by a heteroatom, cycloalkyls optionally having a substituent and optionally interrupted by a heteroatom, and aromatic groups optionally having a substituent and optionally having a heteroatom; and
   R³ and R⁴ are each independently selected from H, alkyls optionally having a substituent and optionally interrupted by a heteroatom, alkenyls optionally having a substituent and optionally interrupted by a heteroatom, alkynyls optionally having a substituent and optionally interrupted by a heteroatom, cycloalkyls optionally having a substituent and optionally interrupted by a heteroatom, and aromatic groups optionally having a substituent and optionally interrupted by a heteroatom, or
   a combination of R³ and R⁴ is selected from oxo groups optionally having a substituent, wherein the oxo groups are formed by the combination of R³ and R⁴ together with the carbon atom to which R³ and R⁴ are bound, or
   a combination of R³ and R⁴ is selected from cycloalkyls optionally having a substituent and optionally interrupted by a heteroatom, wherein the cycloalkyls are formed by the combination of R³ and R⁴ together with the carbon atom to which R³ and R⁴ are bound].
3. The method according to 2, wherein X¹ is O, and X² is O.
4. The method according to 2 or 3, wherein R³ and R⁴ are each independently selected from H, alkyls optionally having a substituent and optionally interrupted by a heteroatom, alkenyls optionally having a substituent and optionally interrupted by a heteroatom, alkynyls optionally having a substituent and optionally interrupted by a heteroatom, cycloalkyls optionally having a substituent and optionally interrupted by a heteroatom, and aromatic groups optionally having a substituent and optionally interrupted by a heteroatom.
5. The method according to 2 or 3, wherein the combination of R³ and R⁴ is selected from cycloalkyls optionally having a substituent and optionally interrupted by a heteroatom, wherein the cycloalkyls are formed by the combination of R³ and R⁴ together with the carbon atom to which R³ and R⁴ are bound.
6. The method according to 5, wherein the cycloalkyls optionally having a substituent and optionally interrupted by a heteroatom, wherein the cycloalkyls are formed by the combination of R³ and R⁴ together with the carbon atom to which R³ and R⁴ are bound, are selected from monocyclic, bicyclic, or tricyclic cycloalkyls.
7. The method according to any one of 2 to 6, wherein
   the aromatic iodonium ylide comprises a solid-phase support, and
   the solid-phase support is a part of a substituent of R¹, R², R³, R⁴, or the combination of R³ and R⁴ in Formula (1) .
8. The method according to 7, wherein the solid-phase support is a solid organic polymer compound.
9. The method according to 8, wherein the solid organic polymer compound is a polystyrene resin.
10. The method according to any one of 7 to 9, wherein
   the aromatic iodonium ylide comprises a linker bound to the solid-phase support, and
   the linker bound to the solid-phase support is a substituent of R¹, R², R³, R⁴, or the combination of R³ and R⁴ in Formula (1), is selected from a group consisting of an alkylene group, a cycloalkylene group, an alkenylene group, an arylene group, a heteroarylene group, a polymethylene group, a polyethylene glycol chain, and a combination thereof, and optionally has at least one of an ether group, an amino group, an amide group, an imide group, an ester group, and a combination thereof.
11. The method according to 10, wherein the linker is selected from a group consisting of an alkylene group, an arylene group, a heteroarylene group, and a combination thereof, and optionally has at least one ether group.
12. The method according to any one of 2 to 11, wherein the aromatic group (Ar) optionally having a substituent and optionally having a heteroatom is selected from an aryl group optionally having a substituent and a heteroaryl group optionally having a substituent.
13. The method according to 12, wherein the heteroaryl group optionally having a substituent is selected from sulfur-containing heteroaryl groups, oxygen-containing heteroaryl groups, nitrogen-containing heteroaryl groups, and heteroaryl groups containing two or more heteroatoms.
14. The method according to any one of 1 to 13, wherein the aromatic astatine compound is represented by the following Formula (2):

   Formula (2): ²¹¹At-Ar

   [wherein, Ar represents an aromatic group optionally having a substituent and optionally having a heteroatom].
15. The method according to any one of 1 to 14, comprising producing astatine using a cyclotron.

### [Advantageous Effects of Invention]

In the aromatic astatine compound production method according to one embodiment of the present invention, since an aromatic iodonium ylide compound is used, nucleophilic astatine that is a relatively safe and simple chemical species can be utilized. In addition, not only it is not necessary to use a transition metal in a raw material of the reaction thereof, but also it is not necessary to use a transition metal as a catalyst; therefore, excellent safety is attained and the resulting reaction product can be relatively simplified. Further, more preferably, the reaction can achieve ²¹¹At labeling in a chemoselective and regioselective manner. Therefore, the aromatic astatine compound production method according to one embodiment of the present invention can be suitably employed for producing a ²¹¹At-labeled aromatic astatine compound (or an aromatic astatine compound labeled by ²¹¹At).

### [Description of Embodiments]

A method of producing an aromatic astatine compound according to one embodiment of the present invention includes allowing an aromatic iodonium ylide to react with astatine to produce the aromatic astatine compound.

As long as an aromatic astatine compound can be produced, there is no limitation on the aromatic iodonium ylide, its production method and the like, and astatine, its production method and the like as well as the conditions and the like of the reaction between the aromatic iodonium ylide and astatine are also not particularly limited.

The aromatic iodonium ylide generally refers to an iodonium ylide compound in which an aromatic group and iodine are directly bound to each other, and any such compound understandable to those skilled in the art can be used as the aromatic iodonium ylide. Examples of the aromatic iodonium ylide include compounds represented by the following Formula (1): 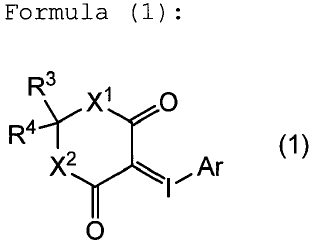 [wherein,
Ar represents an aromatic group optionally having a substituent and optionally having a heteroatom;
X¹ is selected from a group consisting of NR¹, O, and S;
X² is selected from a group consisting of NR², O, and S;
R¹ and R² are each independently selected from H, alkyls optionally having a substituent and optionally interrupted by a heteroatom, cycloalkyls optionally having a substituent and optionally interrupted by a heteroatom, and aromatic groups optionally having a substituent and optionally having a heteroatom; and
R³ and R⁴ are each independently selected from H, alkyls optionally having a substituent and optionally interrupted by a heteroatom, alkenyls optionally having a substituent and optionally interrupted by a heteroatom, alkynyls optionally having a substituent and optionally interrupted by a heteroatom, cycloalkyls optionally having a substituent and optionally interrupted by a heteroatom, and aromatic groups optionally having a substituent and optionally interrupted by a heteroatom, or
a combination of R³ and R⁴ is selected from oxo groups optionally having a substituent, wherein the oxo groups are formed by the combination of R³ and R⁴ together with the carbon atom to which R³ and R⁴ are bound, or
a combination of R³ and R⁴ is selected from cycloalkyls optionally having a substituent and optionally interrupted by a heteroatom, wherein the cycloalkyls are formed by the combination of R³ and R⁴ together with the carbon atom to which R³ and R⁴ are bound].

In one embodiment of the present invention, it is preferred that X¹ is O and X² is O.

In one embodiment of the present invention, X¹ = NR¹ and X² = NR², and R¹ and R² may each be independently selected from H, alkyls optionally having a substituent and optionally interrupted by a heteroatom, cycloalkyls optionally having a substituent and optionally interrupted by a heteroatom, and aromatic groups optionally having a substituent and optionally having a heteroatom.

In one embodiment of the present invention, an "alkyl" refers to a monovalent chain saturated hydrocarbon group, and is not particularly limited as long as an aromatic astatine compound can be obtained. Examples of the alkyl include alkyl groups having, for example, 1 to 24, 1 to 18, 1 to 12, or 1 to 8 carbon atoms (e.g., a methyl group, an ethyl group, an n-propyl group, an isopropyl group, an n-butyl group, an isobutyl group, a tert-butyl group, a pentyl group, a hexyl group, and an octyl group). As long as an aromatic astatine compound can be obtained, the alkyl may have a substituent as appropriate, and may be interrupted by, for example, a heteroatom such as oxygen, nitrogen, or sulfur.

In one embodiment of the present invention, a "cycloalkyl" refers to a monovalent cyclic saturated hydrocarbon group, and is not particularly limited as long as an aromatic astatine compound can be obtained. Examples of the cycloalkyl include cycloalkyl groups having, for example, 3 to 24, 3 to 18, 3 to 12, or 3 to 8 carbon atoms (e.g., a cyclopentyl group, a cyclohexyl group, a cycloheptyl group, and a cyclodecyl group). As long as an aromatic astatine compound can be obtained, the cycloalkyl may have a substituent as appropriate, and may be interrupted by, for example, a heteroatom such as oxygen, nitrogen, or sulfur.

In one embodiment of the present invention, an "aromatic group" refers to a monovalent hydrocarbon group having aromaticity, and is not particularly limited as long as an aromatic astatine compound can be obtained. Generally, the aromatic group can be selected from an aryl group (or aromatic hydrocarbon group) optionally having a substituent and a heteroaryl group (or heteroaromatic group) optionally having a substituent.

Examples of the aryl group (or aromatic hydrocarbon group) optionally having a substituent include a phenyl group, a naphthyl group, a biphenyl group, and a terphenyl group.

Examples of the heteroaryl group (or heteroaromatic group) optionally having a substituent include:
sulfur-containing heteroaryl groups, such as a thiophenyl group (a thiophene group or a thienyl group) and a benzothienyl group;
oxygen-containing heteroaryl groups, such as a furanyl group (or a furan group) and a benzofuranyl group; and
nitrogen-containing heteroaryl groups, such as a pyridyl group (or a pyridine group), a pyrimidinyl group (or a pyrimidine group), a pyrazyl group (or a pyrazine group), a quinolyl group (or a quinoline group), and an isoquinolyl group.

In one embodiment of the present invention, R³ and R⁴ may each be independently selected from H, alkyls optionally having a substituent and optionally interrupted by a heteroatom, alkenyls optionally having a substituent and optionally interrupted by a heteroatom, alkynyls optionally having a substituent and optionally interrupted by a heteroatom, cycloalkyls optionally having a substituent and optionally interrupted by a heteroatom, and aromatic groups optionally having a substituent and optionally having a heteroatom.

With regard to the alkyls optionally having a substituent and optionally interrupted by a heteroatom, the cycloalkyls optionally having a substituent and optionally interrupted by a heteroatom, and the aromatic groups optionally having a substituent and optionally having a heteroatom, reference can be made to the descriptions above.

In one embodiment of the present invention, an "alkynyl" refers to a monovalent hydrocarbon group having a triple bond between carbon atoms, and is not particularly limited as long as an aromatic astatine compound can be obtained. Examples of the alkynyl include alkynyl groups having, for example, 1 to 24, 1 to 18, 1 to 12, or 1 to 8 carbon atoms (e.g., an ethynyl group, a propynyl group, a butynyl group, a pentynyl group, a hexynyl group, and an octynyl group). As long as an aromatic astatine compound can be obtained, the alkynyl may have a substituent as appropriate, and may be interrupted by, for example, a heteroatom such as oxygen, nitrogen, or sulfur.

In one embodiment of the present invention, an "alkenyl" refers to a monovalent hydrocarbon group having a double bond between carbon atoms, and is not particularly limited as long as an aromatic astatine compound can be obtained. Examples of the alkenyl include alkenyl groups having, for example, 1 to 24, 1 to 18, 1 to 12, or 1 to 8 carbon atoms (e.g., an ethenyl group, a propenyl group, a butenyl group, a pentenyl group, a hexenyl group, and an octenyl group). As long as an aromatic astatine compound can be obtained, the alkenyl may have a substituent as appropriate, and may be interrupted by, for example, a heteroatom such as oxygen, nitrogen, or sulfur.

In one embodiment of the present invention, a combination of R³ and R⁴ can be selected from cycloalkyls optionally having a substituent and optionally interrupted by a heteroatom (hereinafter, also referred to as "cycloalkyls (or 1,1-cycloalkylenes) formed by a combination of R³ and R⁴"), wherein the cycloalkyls are formed by the combination of R³ and R⁴ together with the carbon atom to which R³ and R⁴ are bound. The cycloalkyls formed by the combination of R³ and R⁴ are not particularly limited as long as an aromatic astatine compound can be obtained.

The cycloalkyls optionally having a substituent and optionally interrupted by a heteroatom, which is formed by a combination of R³ and R⁴ together with the carbon atom to which R³ and R⁴ are bound, are preferably selected from monocyclic, bicyclic, or tricyclic cycloalkyls.

Examples of the cycloalkyls (or 1,1-cycloalkylenes) formed by the combination of R³ and R⁴ include 1,1-cycloalkylene groups having, for example, 3 to 24, 3 to 18, 3 to 12, 3 to 10, or 4 to 10 carbon atoms. Examples of these 1,1-cycloalkylene groups include: monocyclic cycloalkylene groups, such as a 1,1-cyclopropylene group, a 1,1-cyclobutylene group, a 1,1-cyclopentylene group, a 1,1-cyclohexylene group, a 1,1-cycloheptylene group, and a 1,1-cyclodecylene group; bicyclic cycloalkylene groups, such as a norbornylene group; and tricyclic cycloalkylene groups, such as an adamantylene group.

In one embodiment of the present invention, a "substituent" is not particularly limited as long as an aromatic astatine compound can be obtained.

Examples of the substituent include:
alkyl groups having, for example, 1 to 24, 1 to 18, 1 to 12, or 1 to 8 carbon atoms (e.g., a methyl group, an ethyl group, an n-propyl group, an isopropyl group, an n-butyl group, an isobutyl group, a tert-butyl group, a pentyl group, a hexyl group, and an octyl group);
alkoxy groups having, for example, 1 to 24, 1 to 18, 1 to 12, or 1 to 8 carbon atoms (e.g., a methoxy group, an ethoxy group, an n-propoxy group, an isopropoxy group, an n-butoxy group, an isobutoxy group, a tert-butoxy group, a pentyloxy group, a hexyloxy group, and an octyloxy group);
cycloalkyl groups having, for example, 3 to 24, 3 to 18, 3 to 12, or 3 to 8 carbon atoms (e.g., a cyclopropyl group, a cyclobutyl group, a cyclopentyl group, and a cyclohexyl group);
alkenyl groups having, for example, 1 to 24, 1 to 18, 1 to 12, or 1 to 8 carbon atoms (e.g., an ethenyl group, a propenyl group, a butenyl group, a pentenyl group, a hexenyl group, and an octenyl group);
alkynyl groups having, for example, 1 to 24, 1 to 18, 1 to 12, or 1 to 8 carbon atoms (e.g., an ethynyl group, a propynyl group, a butynyl group, a pentynyl group, a hexynyl group, and an octynyl group);
aryl groups having, for example, 5 to 24, 5 to 18, 5 to 12, or 5 to 8 carbon atoms (e.g., a phenyl group, a naphthyl group, and a biphenyl group);
aryloxy groups having, for example, 5 to 24, 5 to 18, 5 to 12, or 5 to 8 carbon atoms (e.g., a phenoxy group, a naphthyloxy group, and a biphenyloxy group);
heteroaryl groups having, for example, 1 to 24, 1 to 18, 1 to 12, or 1 to 8 carbon atoms (e.g., a thiophenyl group, a furanyl group, a carbazole group, a benzothiophenyl group, a benzofuranyl group, an indolyl group, a pyrrolyl group, a pyridyl group, and a triazole group);
acyl groups having, for example, 1 to 24, 1 to 18, 1 to 12, or 1 to 8 carbon atoms (e.g., an acetyl group, a propionyl group, a butanoyl group, a pentanoyl group, a heptanoyl group, and these acyl groups in which a carbonyl group is substituted with an ester group or an amide group);
amino groups having, for example, 1 to 24, 1 to 18, 1 to 12, or 1 to 8 carbon atoms (e.g., a diphenylamino group and a dimethylamino group); and
fluorine (including a partial fluorine substitution and a complete fluorine substitution), a cyano group, and a nitro group.

These substituents may be cross-linked with each other, or may form a cyclic structure (aromatic group) as a whole. In addition, the above-described substituents may each further include any of the above-described substituents.

As such substituents, alkyl groups, alkoxy groups, cycloalkyl groups, aryl groups, aryloxy groups, heteroaryl groups, and combinations of these groups are preferred.

The aromatic iodonium ylide may include a solid-phase support. The solid-phase support can be a part of a substituent of R¹, R², R³, R⁴, or a combination of R³ and R⁴ in the above-described Formula (1).

More specifically, a substituent that may be contained in, for example, R³, R⁴, an oxo group formed by a combination of R³ and R⁴ together with the carbon atom to which R³ and R⁴ are bound, or a cycloalkyl optionally interrupted by a heteroatom, wherein the cycloalkyl is formed by a combination of R³ and R⁴ together with the carbon atom to which R³ and R⁴ are bound, can have a solid-phase support.

In addition, for example, when X¹ is selected from NR¹ or X² is selected from NR², a substituent that may be contained in R¹ and R² (more specifically, for example, an alkyl optionally interrupted by a heteroatom, a cycloalkyl optionally interrupted by a heteroatom, or an aromatic group optionally having a heteroatom) can have a solid-phase support.

Therefore, the method of producing an aromatic astatine compound according to one embodiment of the present invention includes allowing an aromatic iodonium ylide supported on a solid phase (or having a solid-phase support) to react with astatine. The use of an aromatic iodonium ylide supported on a solid phase can exert advantageous effects of, for example, making leakage of astatine, which is a radioactive compound, less likely to occur and improving the safety in handling of astatine since such an aromatic iodonium ylide reacts in a solid phase and thus can be purified by filtration or the like and handled in a closed system in which a post-treatment is more easily performed.

The term "solid-phase support" used herein refers to a solid-phase moiety which can exist as a part of a substituent of an aromatic iodonium ylide and immobilize the aromatic iodonium ylide, and it is not particularly limited as long as the target aromatic astatine compound of the present invention can be obtained. When an aromatic iodonium ylide has a solid-phase support in its substituent, the aromatic iodonium ylide may also be referred to as "solid phase-supported aromatic iodonium ylide".

Examples of the solid-phase support include solid organic polymer compounds, solid inorganic compounds, and solid complexes composed of an organic polymer compound and an inorganic compound.

More specific examples include the following compounds (basic skeletons):
solid organic polymer compounds, such as polystyrene resins (e.g., resins containing polystyrene as a basic skeleton, for example, so-called polystyrenes, polystyrene/divinylbenzene resins, and polyethylene glycol-polystyrene/divinylbenzene resins), polyacrylamide resins, PEGA resins, celluloses, polyesters, and polyamides;
solid inorganic compounds, such as silica gel, alumina, and graphite; and
solid complexes formed by a combination of an organic compound and an inorganic compound.

A solid organic polymer compound composed of an organic compound is preferred and a polystyrene resin is preferred.

The above-exemplified solid-phase supports are commercially available, or can be synthesized by a known method or a method similar thereto. Examples of a synthetic intermediate having such a solid-phase support include polystyrene resins in which a halogen group is introduced to a benzene ring via an alkylene group such as a methylene group or an ethylene group.

When the aromatic iodonium ylide has a solid-phase support, the aromatic iodonium ylide also has a linker bound to the solid-phase support, and this linker bound to the solid-phase support may be a substituent of R¹, R², R³, R⁴, or a combination of R³ and R⁴ in the above-described Formula (1). The linker may be a substituent moiety that can exist between the solid-phase support and the aromatic iodonium ylide. Such a substituent moiety (linker) is not particularly limited as long as the target aromatic astatine compound of the present invention can be obtained.

Examples of the substituent moiety (linker) include the followings.

When the solid-phase support is a polystyrene resin, a substituent moiety that is bound to a benzene ring contained in the basic skeleton of the polystyrene resin and exists between the polystyrene resin and the aromatic iodonium ylide can be equivalent to a linker. Such a substituent moiety may be, for example, a divalent group obtained by further removing a hydrogen atom from any of the above-described substituents, and examples thereof include: an alkylene group, a cycloalkylene group, an alkenylene group, an alkynylene group, an arylene group, a heteroarylene group, a polymethylene group, structures containing an oxygen atom and other heteroatom such as a polyethylene glycol chain, and combinations thereof. These divalent groups may further contain, for example, at least one of an ether group, a thioether group, an amino group, an amide group, an imide group, an ester group, and a combination of these groups. The substituent moiety may further contain a moiety of the above-described substituents.

The aromatic iodonium ylide includes a linker bound to the solid-phase support, and the linker bound to the solid-phase support is a substituent of R¹, R², R³, R⁴, or a combination of R³ and R⁴ in Formula (1), and selected from a group consisting of an alkylene group, a cycloalkylene group, an alkenylene group, an alkynylene group, an arylene group, a heteroarylene group, a polymethylene group, a polyethylene glycol chain, and a combination thereof. Further, the linker may include at least one of an ether group, a thioether group, an amino group, an amide group, an imide group, an ester group, and a combination of these groups.

It is preferred that the linker be selected from a group consisting of an alkylene group, an arylene group, a heteroarylene group, and a combination thereof, and optionally have at least one ether group.

When the aromatic iodonium ylide has a solid-phase support, more specific examples of the substituent moiety (linker) bound to the solid-phase support include those represented by the following Formula (3):

Formula (3): (solid-phase support)-alkylene-Y¹-alkylene-Y²-(R¹, R², R³, R⁴, or a combination of R³ and R⁴)

[wherein, the solid-phase support is as described above; R¹ to R⁴ are as described above for Formula (1); Y¹ and Y² are each independently selected from a group consisting of an alkylene group, a cycloalkylene group, an alkenylene group, an alkynylene group, an arylene group, a heteroarylene group, a polymethylene group, and a polyethylene glycol chain, and optionally have one selected from an ether group, a thioether group, an amino group, an amide group, an imide group, an ester group; and it is preferred that Y¹ and Y² are each independently selected from the group consisting of an alkylene group, an arylene group, and a heteroarylene group, and optionally have an ether group].

One embodiment of the present invention can provide a solid phase-supported aromatic iodonium ylide (or an aromatic iodonium ylide including a solid-phase support), which can be represented by the following Formula (1): 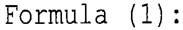 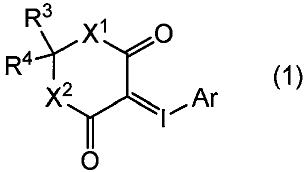 [wherein,
Ar represents an aromatic group optionally having a substituent and optionally having a heteroatom;
X¹ is selected from a group consisting of NR¹, O, and S;
X² is selected from a group consisting of NR², O, and S;
R¹ and R² are each independently selected from H, alkyls optionally having a substituent and optionally interrupted by a heteroatom, cycloalkyls optionally having a substituent and optionally interrupted by a heteroatom, and aromatic groups optionally having a substituent and optionally having a heteroatom;
R³ and R⁴ are each independently selected from H, alkyls optionally having a substituent and optionally interrupted by a heteroatom, alkenyls optionally having a substituent and optionally interrupted by a heteroatom, alkynyls optionally having a substituent and optionally interrupted by a heteroatom, cycloalkyls optionally having a substituent and optionally interrupted by a heteroatom, and aromatic groups optionally having a substituent and optionally interrupted by a heteroatom, or
a combination of R³ and R⁴ is selected from oxo groups optionally having a substituent, wherein the oxo groups are formed by the combination of R³ and R⁴ together with the carbon atom to which R³ and R⁴ are bound, or
a combination of R³ and R⁴ is selected from cycloalkyls optionally having a substituent and optionally interrupted by a heteroatom, wherein the cycloalkyls are formed by the combination of R³ and R⁴ together with the carbon atom to which R³ and R⁴ are bound; and
R¹, R², R³, R⁴, the oxo group optionally having a substituent, the oxo group being formed by a combination of R³ and R⁴ together with the carbon atom to which R³ and R⁴ are bound, or a cycloalkyl optionally having a substituent and optionally interrupted by a heteroatom, the cycloalkyl being formed by a combination of R³ and R⁴ together with the carbon atom to which R³ and R⁴ are bound, optionally includes a solid-phase support as a part of a substituent thereof].

With regard to the X¹, R¹, X², R², R³, R⁴, substituent and the like, reference can be made to the descriptions above.

In one embodiment of the present invention, Ar in the above-described Formula (1) is an aromatic group optionally having a substituent and optionally having a heteroatom, and it is not particularly limited as long as an aromatic astatine compound can be obtained.

In one embodiment of the present invention, regarding Ar, the aromatic group optionally having a substituent and optionally having a heteroatom can be selected from an aryl group (or aromatic hydrocarbon group) optionally having a substituent and a heteroaryl group (or heteroaromatic group) optionally having a substituent.

Regarding Ar, examples of the aryl group (or aromatic hydrocarbon group) optionally having a substituent include a phenyl group, a naphthyl group, an anthracenyl group (or an anthracene group), a phenanthrenyl group (or a phenanthrene group), a biphenyl group, a terphenyl group, a pyrenyl group (or a pyrene group), a perylenyl group (or a perylene group), and a triphenylenyl group (or a triphenylene group). The structures of these groups are represented by the following chemical formulae (wherein, one hydrogen atom bound to any of the carbon atoms is removed):

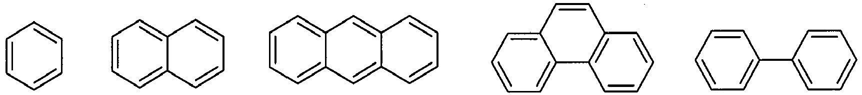

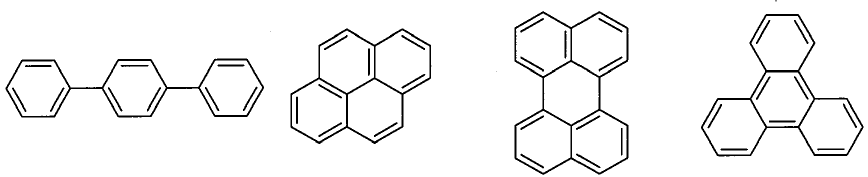

Regarding Ar, examples of the heteroaryl group (or heteroaromatic group) optionally having a substituent include sulfur-containing heteroaryl groups, oxygen-containing heteroaryl groups, nitrogen-containing heteroaryl groups, heteroaryl groups containing two or more heteroatoms (e.g., nitrogen and sulfur).

Examples of the sulfur-containing heteroaryl groups include a thiophenyl group (a thiophene group or a thienyl group), a benzothiophene group, a dibenzothiophene group, a phenylthiophene group, and a diphenylthiophene group. The structures of these groups are represented by the following chemical formulae (wherein, one hydrogen atom bound to any of the carbon atoms is removed):

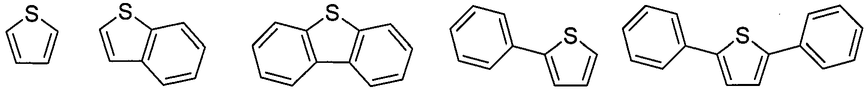

Examples of the oxygen-containing heteroaryl groups include a furanyl group (or a furan group), a benzofuranyl group, a dibenzofuranyl group, a phenylfuran group, a diphenylfuran group, a chromen-4-one group, and a chromen-2-one group. The structures of these groups are represented by the following chemical formulae (wherein, one hydrogen atom bound to any of the carbon atoms is removed):

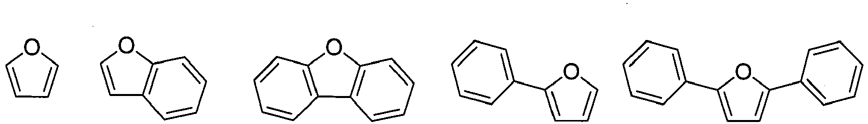

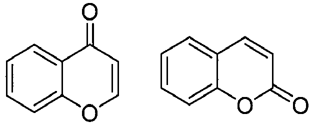

Examples of the nitrogen-containing heteroaryl groups include a pyridyl group (or a pyridine group), a pyrimidinyl group (or a pyrimidine group), a pyrazyl group (or a pyrazine group), a quinolyl group (or a quinoline group), an isoquinolyl group (or a isoquinoline group), a carbazolyl group (or a carbazole group), a 9-phenylcarbazolyl group, an acridinyl group (or an acridine group), a quinazolyl group (or a quinazoline group), a quinoxalyl group (or a quinoxaline group), a 1,6-naphthyridinyl group, a 1,8-naphthyridinyl group, a porphyrin group (or a porphyrin ring), a pyridazine group, a 1,3,5-triazine group, a pyrazole group, a 1,2,4-triazole group, a 1,2,3-triazole group, an indole group, a benzimidazole group, an indazole group, a benzo[d][1,2,3]triazole group, a pyrrolo[2,3-b]pyridine group, an imidazo[4,5-b]pyridine group, a pyrrolo[3,2-c]pyridine group, an imidazo[4,5-c]pyridine group, a pyrrolo[2,3-d]pyrimidine group, and a purine group. The structures of these groups are represented by the following chemical formulae (wherein, one hydrogen atom bound to any of the carbon or nitrogen atoms is removed):

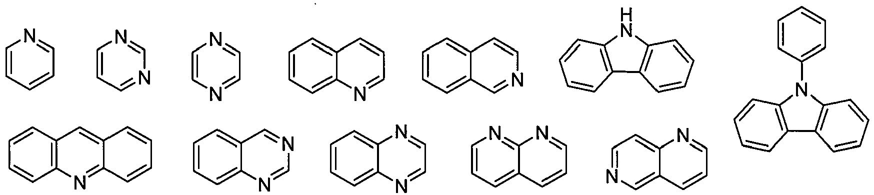

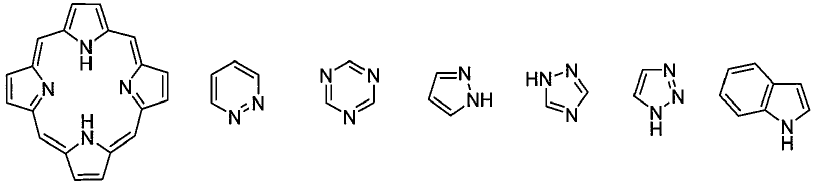

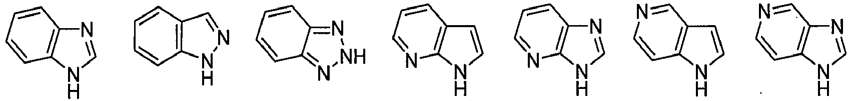

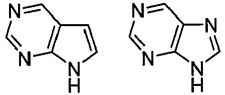

Examples of the heteroaryl groups containing two or more heteroatoms (e.g., nitrogen and oxygen, nitrogen and sulfur, or oxygen and sulfur) include a quinolin-4-one group, a thiochromen-4-one group, a quinolin-2-one group, a thiochromen-2-one group, a phthalazin-1-one group, an isoxazole group, an isothizole group, a 1,2,4-oxadiazole group, a 1,2,4-thiadiazole group, a 1,2,3-oxadiazole group, a 1,2,3-thiadiazole group, a benzoxazole group, a benzothizole group, a benzoisoxazole group, a benzoisothizole group, a benzo[c]isoxazole group, a benzo[c]isothizole group, a benzo[c][1,2,5]oxadiazole group, a benzo[c][1,2,5]thiadiazole group, an oxazolo[5,4-b]pyridine group, a thiazolo[5,4-b]pyridine group, an oxazolo[4,5-c]pyridine group, a thiazolo[4,5-c]pyridine group, an oxazolo[5,4-d]pyrimidine group, and a thiazolo[5,4-d]pyrimidine group. The structures of these groups are represented by the following chemical formulae (wherein, one hydrogen atom bound to any of the carbon or nitrogen atoms is removed):

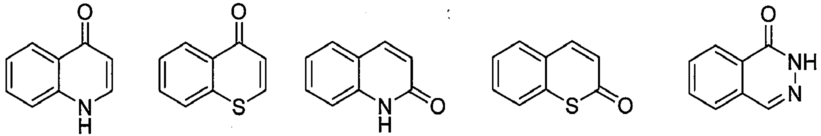

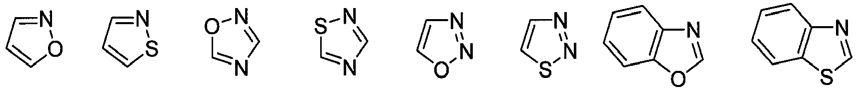

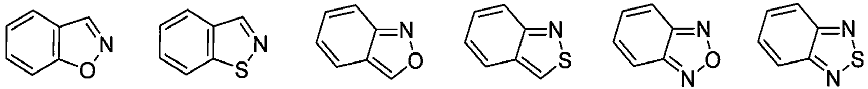

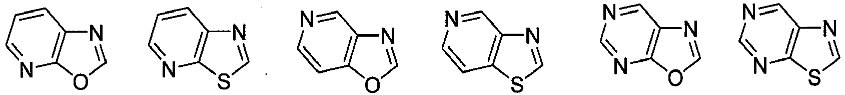

Regarding Ar, a substituent that may be contained in the aryl group and the heteroaryl group is not particularly limited as long as the target aromatic astatine compound of the present invention can be obtained.

Examples of the substituent include:
alkyl groups having, for example, 1 to 24, 1 to 18, 1 to 12, or 1 to 8 carbon atoms (e.g., a methyl group, an ethyl group, an n-propyl group, an isopropyl group, an n-butyl group, an isobutyl group, a tert-butyl group, a pentyl group, a hexyl group, and an octyl group);
alkoxy groups having, for example, 1 to 24, 1 to 18, 1 to 12, or 1 to 8 carbon atoms (e.g., a methoxy group, an ethoxy group, an n-propoxy group, an isopropoxy group, an n-butoxy group, an isobutoxy group, a tert-butoxy group, a pentyloxy group, a hexyloxy group, and an octyloxy group);
cycloalkyl groups having, for example, 3 to 24, 3 to 18, 3 to 12, or 3 to 8 carbon atoms (e.g., a cyclopropyloxy group, a cyclobutyloxy group, a cyclopentyloxy group, and a cyclohexyloxy group);
alkenyl groups having, for example, 1 to 24, 1 to 18, 1 to 12, or 1 to 8 carbon atoms (e.g., an ethenyl group, a propenyl group, a butenyl group, a pentenyl group, a hexenyl group, and an octenyl group);
alkynyl groups having, for example, 1 to 24, 1 to 18, 1 to 12, or 1 to 8 carbon atoms (e.g., an ethynyl group, a propynyl group, a butynyl group, a pentynyl group, a hexynyl group, and an octynyl group);
aryl groups having, for example, 5 to 24, 5 to 18, 5 to 12, or 5 to 8 carbon atoms (e.g., a phenyl group, a naphthyl group, and a biphenyl group);
aryloxy groups having, for example, 5 to 24, 5 to 18, 5 to 12, or 5 to 8 carbon atoms (e.g., a phenoxy group, a naphthyloxy group, and a biphenyloxy group);
heteroaryl groups having, for example, 4 to 24, 1 to 18, 1 to 12, or 1 to 8 carbon atoms (e.g., a thiophenyl group, a furanyl group, a carbazole group, a benzothiophenyl group, a benzofuranyl group, an indolyl group, a pyrrolyl group, and a pyridyl group);
acyl groups having, for example, 1 to 24, 1 to 18, 1 to 12, or 1 to 8 carbon atoms (e.g., an acetyl group, a propionyl group, a butanoyl group, a pentanoyl group, a heptanoyl group, and these acyl groups in which a carbonyl group is substituted with an ester group or an amide group);
amino groups having, for example, 1 to 24, 1 to 18, 1 to 12, or 1 to 8 carbon atoms (e.g., a diphenylamino group and a dimethylamino group); and
fluorine (including a partial fluorine substitution and a complete fluorine substitution), a cyano group, and a nitro group.

These substituents may be cross-linked with each other, or may form a cyclic structure (a ring-fused structure or a cross-linked structure) as a whole. In addition, the above-described substituents may each further include any of the above-described substituents.

The above-described aromatic iodonium ylide can be produced by a known method. This production method is not particularly limited as long as an aromatic astatine compound can be obtained. With regard to the production method, reference can be made to, for example, Non-patent Literature 3: Benjamin H. Rotstein, Nickeisha A. Stephenson, Neil Vasdev and Steven H. Liang, Nature Communications 2014, 4365, Non-patent Literature 4: Keitaro Matsuoka, Narumi Komami, Masahiro Kojima, Tatsuhiko Yoshino, and Shigeki Matsunaga, Asian J. Org. Chem. 2019, 8, 1107-1110, and Non-patent Literature 5: Narumi Komami, Keitaro Matsuoka, Ayako Nakano, Masahiro Kojima, Tatsuhiko Yoshino, and Shigeki Matsunaga, Chem. Eur. J. 2019, 25, 1217-1220).

An aromatic iodonium ylide having a solid-phase support can be produced by appropriately employing a known method. A more concrete example thereof is represented by the chemical formula shown below. It is noted here that the chemical formula shown below represents a case where the solid-phase support is bound to R⁴; however, the solid-phase support may be bound to R³ or a combination of R³ and R⁴ and, when X¹ or X² is N, the solid-phase support may be bound to R¹ or R². For example, an aromatic precursor (11) can be obtained by a combination of a germylation reaction (Synthesis 2018, 50, 206) and a direct hypervalent iodine introduction reaction (Chem. Eur. J. 2019, 25, 1217; Asian J. Org. Chem. 2019, DOI: 10.1002/ajoc.201900200). Further, a Meldrum's acid precursor (12) having a solid-phase support can be obtained by allowing a Meldrum's acid unit and the solid-phase support (e.g., a solid organic polymer compound) to bind with each other by utilizing an appropriate substituent. The Meldrum's acid precursor (12) having the solid-phase support and the aromatic precursor (11) are allowed to react with each other, whereby an aromatic iodonium ylide (1) having the solid-phase support can be produced.

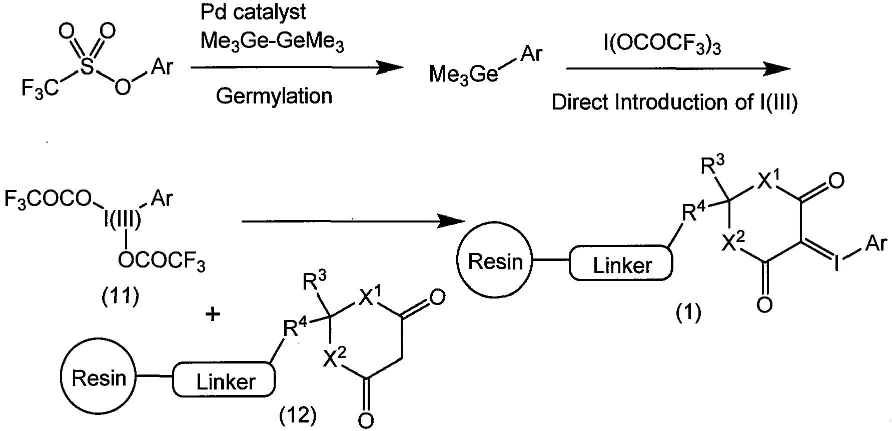

In one embodiment of the present invention, astatine can be produced by a known method. This production method is not particularly limited as long as an aromatic astatine compound can be obtained.

Astatine can be produced using a cyclotron. With regard to a method of producing astatine using a cyclotron, reference can be made to Non-patent Literature 6: Kotaro Nagatsu, Katsuyuki Minegishi, Masami Fukuda, Hisashi Suzuki, Sumitaka Hasegawa, Ming-Rong Zhang, "Production of 211At by a vertical beam irradiation method", Applied Radiation and Isotopes, 94 (2014) 363-371. For example, ²¹¹At can be used in a form of a ²¹¹At solution that is obtained by isolating and purifying ²¹¹At nuclide (or ²¹¹At) produced by a cyclotron and dissolving the ²¹¹At in a solvent, for example, a halogen-based solvent such as CHCl₃, an amide-based solvent such as DMF, a sulfoxide-based solvent such as DMSO, an alcohol-based solvent such as MeOH, a ketone-based solvent, or an ether-based solvent.

In one embodiment of the present invention, an aromatic astatine compound can be produced by allowing an aromatic iodonium ylide to react with astatine. One example of this reaction is represented by the reaction formula shown below. In one embodiment of the present invention, an "aromatic astatine compound" refers to a compound in which astatine is directly bound to an aromatic group such as a heteroaryl group or an aryl group. 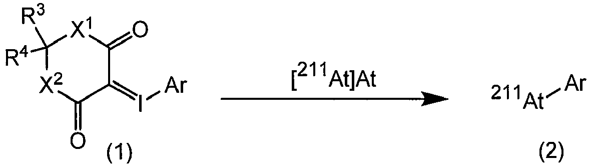 [wherein, with regard to X¹, X², R³, R⁴, and Ar, reference can be made to the descriptions above relating to aromatic iodonium ylide]

In one embodiment of the present invention, an aromatic astatine compound can also be produced by allowing an aromatic iodonium ylide having a solid-phase support to react with astatine. One example of this reaction is represented by the reaction formula shown below. It is noted here that the chemical formula shown below represents a case where the solid-phase support is bound to R⁴; however, the solid-phase support may be bound to R³ or a combination of R³ and R⁴ and, when X¹ or X² is N, the solid-phase support may be bound to R¹ or R². 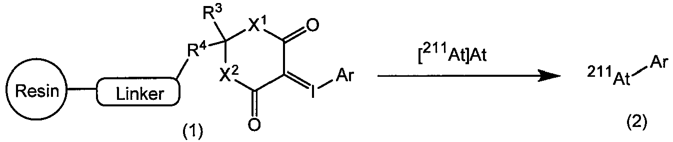 [wherein, with regard to X¹, X², R³, R⁴, and Ar, reference can be made to the descriptions above relating to aromatic iodonium ylide]

In one embodiment of the present invention, the reaction between an aromatic iodonium ylide and astatine is not particularly limited in terms of a method, conditions and the like thereof, as long as an aromatic astatine compound can be produced.

For example, an aromatic iodonium ylide and ²¹¹At are allowed to react (in a solution state) by mixing them in, for example, the above-described organic solvent. A reaction concentration, a reaction temperature, and a reaction time can be selected as appropriate. For the reaction, various additives can be used as appropriate. As the additives, for example, a phase transfer catalyst such as an alkyl ammonium salt, a reducing agent such as a sulfite, and a base such as a Lewis base or a Bronsted base are allowed to exist as appropriate.

More specifically, for example, the ²¹¹At solution is added to a reaction vial, and the solvent is evaporated to dryness by heating to, for example, 55°C, while blowing an inert gas (e.g., nitrogen gas) thereto. Subsequently, for example, a DMF solution that contains, for example, an aryliodonium ylide 1, Et₄NHCO₃, and PPh₃ is added to the ²¹¹At at room temperature. The resulting mixture is allowed to react, for example, in a nitrogen atmosphere at 100°C for 30 minutes. A portion of the resulting reaction solution can be taken out and analyzed by an analysis means, such as Radio-HPLC or Radio-TLC, to calculate the radiochemical yield of a target ²¹¹At-labeled compound (or ²¹¹At-labeled form) .

The reaction temperature of ²¹¹At and the aryliodonium ylide may be, for example, 0°C or higher, 5°C or higher, 10°C or higher, 15°C or higher, 20°C or higher, 40°C or higher, or 60°C or higher. The reaction temperature of ²¹¹At and the aryliodonium ylide may be, for example, 180°C or lower, 170°C or lower, 160°C or lower, 140°C or lower, or 120°C or lower.

The reaction time of ²¹¹At and the aryliodonium ylide may be, for example, 1 minute or longer, 2 minutes or longer, 5 minutes or longer, 10 minutes or longer, 20 minutes or longer, 40 minutes or longer, 1 hour or longer, or 2 hours or longer. The reaction time of ²¹¹At and the aryliodonium ylide may be, for example, 15 hours or shorter, 10 hours or shorter, 7 hours or shorter, 5 hours or shorter, or 3 hours or shorter.

With regard to a method, conditions and the like of the above-described reaction between an aromatic iodonium ylide and astatine, when the aromatic iodonium ylide has a solid-phase support, reference can be made to the descriptions above relating the reaction between such an aromatic iodonium ylide and astatine.

One embodiment of the present invention can provide an apparatus for producing an aromatic astatine compound, which includes a reaction section where an aromatic iodonium ylide and astatine are allowed to react.

In addition, an apparatus for producing an aromatic astatine compound, which includes a reaction section where an aromatic iodonium ylide having a solid-phase support and astatine are allowed to react, can be provided as well.

With regard to a method, conditions and the like of the reaction between an aromatic iodonium ylide and astatine, reference can be made to the descriptions relating to these apparatuses for producing an aromatic astatine compound.

In one embodiment of the present invention, an aromatic astatine compound can be represented by, for example, the following Formula (2):

Formula (2): ²¹¹At-Ar

[wherein, Ar represents an aromatic group optionally having a substituent and optionally having a heteroatom].

With regard to Ar in Formula (2), reference can be made to the descriptions of Ar relating to the above-described aromatic iodonium ylide (1).

In the aromatic astatine compound production method according to one embodiment of the present invention, since an aromatic iodonium ylide is used, it is believed that nucleophilic astatine, which is a relatively safe and simple chemical species, can be utilized. In addition, not only it is not necessary to use a transition metal in a raw material of the reaction thereof, but also it is not necessary to use a transition metal as a catalyst. Thus, excellent safety is attained and the resulting product can be simplified. Further, more preferably, ²¹¹At labeling can be achieved in a chemoselective and regioselective manner. Therefore, the aromatic astatine compound production method according to one embodiment of the present invention can be suitably employed for producing a variety of ²¹¹At-labeled aromatic astatine compounds.

### [Examples]

The present invention will now be described more concretely and in more detail by way of Examples and Comparative Examples; however, the below-described Examples merely represent one mode of the present invention, and the present invention is not limited by these Examples at any rate.

### (General Production Procedures)

As Examples that embody the reaction of the present invention, general production procedures are described below in detail regarding the synthesis of a ²¹¹At-labeled arene 2 based on the following reaction using [²¹¹At]astatine and an aryliodonium ylide 1.

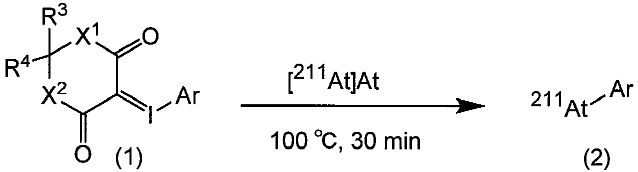

²¹¹At nuclei were produced by nuclear reaction of ²⁰⁹Bi(a,2n)²¹¹At using a cyclotron (930-type AVF Cyclotron (product name), manufactured by Sumitomo Heavy Industries, Ltd.). The thus obtained ²¹¹At nuclei were isolated and purified, and then dissolved in CHCl₃ to use ²¹¹At in the form of a CHCl₃ solution. With regard to a production method of ²¹¹At, reference can be made to Non-patent Literature 6: Kotaro Nagatsu, Katsuyuki Minegishi, Masami Fukuda, Hisashi Suzuki, Sumitaka Hasegawa, Ming-Rong Zhang, "Production of 211At by a vertical beam irradiation method", Applied Radiation and Isotopes, 94 (2014), 363-371.

The ²¹¹At/CHCl₃ solution was added to a reaction vial and heated to 55°C while blowing nitrogen gas into the reaction vial so as to evaporate the solvent to dryness. Subsequently, a DMF solution (500 µL) of the aryliodonium ylide 1 (10 mg), Et₄NHCO₃ (7 mg, 37 µmol) and PPh₃ (5 mg, 19 µmol) was added at room temperature. The thus obtained reaction solution was allowed to react in a nitrogen atmosphere at 100°C for 30 minutes. A portion of this reaction solution was taken out and analyzed by Radio-HPLC and Radio-TLC. From the results of the Radio-TLC analysis, the radiochemical yield of a target ²¹¹At-labeled compound 2 was calculated. For the Radio-HPLC analysis, a 4.6 mm (inner diameter) × 150 mm (length) column with a silica gel having a particle size of 5 µm, InertSustain C18 manufactured by GL Sciences Inc., was used. For the Radio-TLC analysis, a TLC plate was used,

### (Example 1)

### Production of (8R,9S,13S,14S)-3-(astato-²¹¹At)-13-methyl-6,7,8,9,11,12,13,14,15,16-decahydro-17H-cyclopenta[a]phenanthren-17-one (2a)

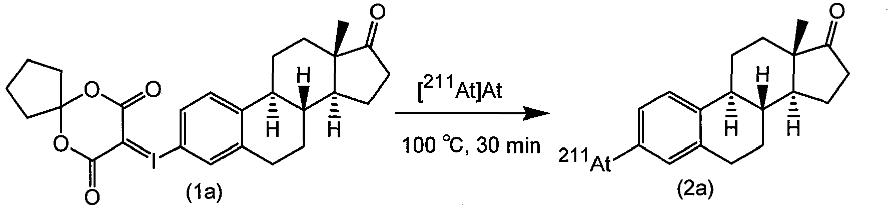

As a result of carrying out a reaction using a ²¹¹At/CHCl₃ solution (43 MBq) and an aryliodonium ylide 1a (10 mg, 18 µmol), a target ²¹¹At-labeled compound 2a was obtained with a Radio-TLC analysis radiochemical yield of 92%. The aryliodonium ylide 1a was produced referring to Non-patent Literature 5.

Radio-HPLC analysis conditions: eluent = MeCN:HCOOH 0.1% aqueous solution = 70:30, column temperature = 25°C, flow rate = 1 mL/min, retention time of ²¹¹At-labeled compound 2a = 11 to 14 minutes (peak top: 12.5 minutes)

Radio-TLC analysis conditions: developing solvent = hexane/AcOEt = 5:1, Rf value of ²¹¹At-labeled compound 2a = 0.46

### (Example 2)

### Production of 2-(4-(4-(astato-²¹¹At)phenyl)4-oxobutyl)isoindoline-1,3-dione (2b)

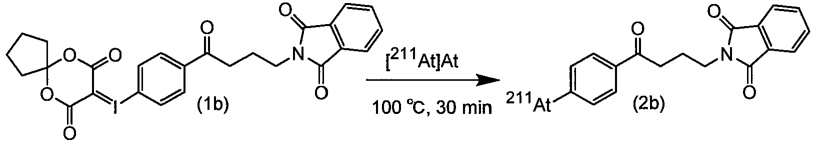

As a result of carrying out a reaction using a ²¹¹At/CHCl₃ solution (17 MBq) and an aryliodonium ylide 1b (10 mg, 17 µmol), a target ²¹¹At-labeled compound 2b was obtained with a Radio-TLC analysis radiochemical yield of 99%. The aryliodonium ylide 1b was produced referring to Non-patent Literature 5.

Radio-HPLC analysis conditions: eluent = MeCN:HCOOH 0.1% aqueous solution = 55:45, column temperature = 25°C, flow rate = 1 mL/min, retention time of ²¹¹At-labeled compound 2b = 8 to 13 minutes (peak top: 10.0 minutes)

Radio-TLC analysis conditions: developing solvent = hexane/AcOEt = 3:1, Rf value of ²¹¹At-labeled compound 2b = 0.32

### (Example 3)

### Production of methyl(S)-3-(4-(astato-²¹¹At)phenyl)-2-((tert-butoxycarbonyl)amino)propanoate (2c)

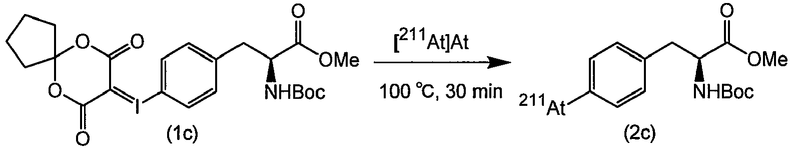

As a result of carrying out a reaction using a ²¹¹At/CHCl₃ solution (18 MBq) and an aryliodonium ylide 1c (10 mg, 17 µmol), a target ²¹¹At-labeled compound 2c was obtained with a Radio-TLC analysis radiochemical yield of 74%. The aryliodonium ylide 1c was produced referring to Non-patent Literature 5.

Radio-HPLC analysis conditions: eluent = MeCN:HCOOH 0.1% aqueous solution = 55:45, column temperature = 25°C, flow rate = 1 mL/min, retention time of ²¹¹At-labeled compound 2c = 7 to 12 minutes (peak top: 9.5 minutes)

Radio-TLC analysis conditions: developing solvent = hexane/AcOEt = 3:1, Rf value of ²¹¹At-labeled compound 2c = 0.45

### (Example 4)

Production of (8R,9S,13S,14S)-3-(astato-²¹¹At)-13-methyl-6,7,8,9,11,12,13,14,15,16-decahydro-17H-cyclopenta[a]phenanthren-17-one (2a)

A ²¹¹At-labeled compound 2a was obtained with a Radio-TLC analysis radiochemical yield of 32% by the same method as described in Example 1, except that a ²¹¹At/CHCl₃ solution (15 MBq) was used in place of the ²¹¹At/CHCl₃ solution (43 MBq), and MeOH was used in place of DMF.

### (Example 5)

Production of (8R,9S,13S,14S)-3-(astato-²¹¹At)-13-methyl-6,7,8,9,11,12,13,14,15,16-decahydro-17H-cyclopenta[a]phenanthren-17-one (2a)

A ²¹¹At-labeled compound 2a was obtained with a Radio-TLC analysis radiochemical yield of 50% by the same method as described in Example 1, except that a ²¹¹At/CHCl₃ solution (53 MBq) was used in place of the ²¹¹At/CHCl₃ solution (43 MBq), and DMSO was used in place of DMF.

### (Example 6)

Production of (8R,9S,13S,14S)-3-(astato-²¹¹At)-13-methyl-6,7,8,9,11,12,13,14,15,16-decahydro-17H-cyclopenta[a]phenanthren-17-one (2a)

A ²¹¹At/CHCl₃ solution (52 MBq) was heated to 55°C while blowing nitrogen gas thereto so as to evaporate solvent to dryness. The resultant was dissolved in MeOH (30 µL) and added to a reaction vial. Subsequently, the aryliodonium ylide 1a (2 mg, 3.6 µmol), Et₄NHCO₃ (7 mg, 37 µmol), and MeOH (70 µL) were added to the reaction vial at room temperature. The thus obtained reaction solution was allowed to react in a nitrogen atmosphere at 100°C for 30 minutes. A portion of this reaction solution was taken out and analyzed by Radio-HPLC and Radio-TLC, as a result of which it was found that a target ²¹¹At-labeled compound 2a was obtained with a Radio-TLC analysis radiochemical yield of 27%.

### (Example 7)

Production of (8R,9S,13S,14S)-3-(astato-²¹¹At)-13-methyl-6,7,8,9,11,12,13,14,15,16-decahydro-17H-cyclopenta[a]phenanthren-17-one (2a)

A ²¹¹At-labeled compound 2a was obtained with a Radio-TLC analysis radiochemical yield of 17% by the same method as described in Example 6, except that a ²¹¹At/CHCl₃ solution (32 MBq) was used in place of the ²¹¹At/CHCl₃ solution (43 MBq), and an aqueous sodium sulfite solution (40 mg/mL, 10 µL) was newly added as an additive.

### (Example 8)

### Production of ethyl 2-(4-(astato-²¹¹At)phenoxy)-2-methyl propanoate (2d)

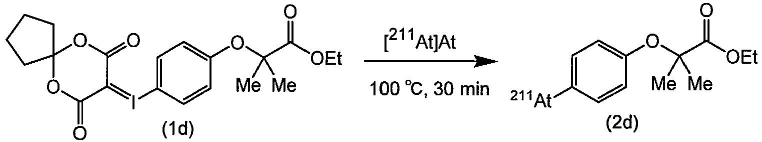

As a result of carrying out a reaction using a ²¹¹At/CHCl₃ solution (34 MBq) and an aryliodonium ylide 1d (10 mg, 20 µmol), a target ²¹¹At-labeled compound 2d was obtained with a Radio-TLC analysis radiochemical yield of 63%. The aryliodonium ylide 1d was produced referring to Non-patent Literature 5.

Radio-HPLC analysis conditions: eluent = MeCN:HCOOH 0.1% aqueous solution = 60:40, column temperature = 25°C, flow rate = 1 mL/min, retention time of ²¹¹At-labeled compound 2d = 12.2 to 13.6 minutes (peak top: 12.8 minutes)

Radio-TLC analysis conditions: developing solvent = hexane/AcOEt = 8:1, Rf value of ²¹¹At-labeled compound 2d = 0.50

### (Example 9)

### Production of 6-(astato-²¹¹At)quinoline (2e)

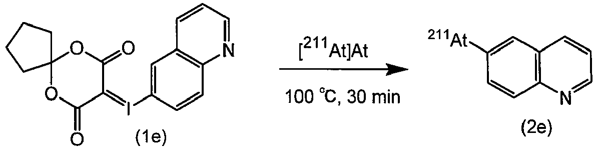

As a result of carrying out a reaction using a ²¹¹At/CHCl₃ solution (24 MBq) and an aryliodonium ylide 1e (10 mg, 24 µmol), a target ²¹¹At-labeled compound 2e was obtained with a Radio-TLC analysis radiochemical yield of 99%. The aryliodonium ylide 1e was produced referring to Non-patent Literature 4.

Radio-HPLC analysis conditions: eluent = MeCN:HCOOH 0.1% aqueous solution = 30:70, column temperature = 25°C, flow rate = 1 mL/min, retention time of ²¹¹At-labeled compound 2e = 6.8 to 7.8 minutes (peak top: 7.3 minutes)

Radio-TLC analysis conditions: developing solvent = hexane/AcOEt = 1:1, Rf value of ²¹¹At-labeled compound 2e = 0.50

### (Example 10)

### Production of 5-(astato-²¹¹At)benzo[b]thiophene (2f)

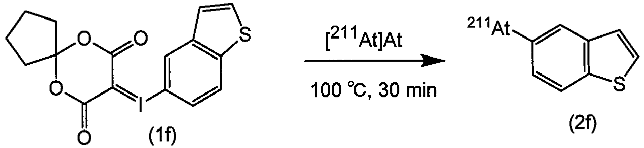

As a result of carrying out a reaction using a ²¹¹At/CHCl₃ solution (31 MBq) and an aryliodonium ylide 1f (10 mg, 23 µmol), a target ²¹¹At-labeled compound 2f was obtained with a Radio-TLC analysis radiochemical yield of 95%. The aryliodonium ylide 1f was produced referring to Non-patent Literature 4.

Radio-HPLC analysis conditions: eluent = MeCN:HCOOH 0.1% aqueous solution = 60;40, column temperature = 25°C, flow rate = 1 mL/min, retention time of ²¹¹At-labeled compound 2f = 13.4 to 14.6 minutes (peak top: 13.9 minutes)

Radio-TLC analysis conditions: developing solvent = hexane, Rf value of ²¹¹At-labeled compound 2f = 0.43

### (Example 11)

### Production of 5-(astato-²¹¹At)-3-methylbenzo[d]isoxazole (2g)

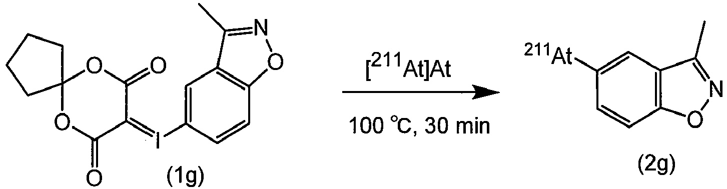

As a result of carrying out a reaction using a ²¹¹At/CHCl₃ solution (28 MBq) and an aryliodonium ylide 1g (10 mg, 24 µmol), a target ²¹¹At-labeled compound 2g was obtained with a Radio-TLC analysis radiochemical yield of 94%. The aryliodonium ylide 1g was produced referring to Non-patent Literature 5.

Radio-HPLC analysis conditions: eluent = MeCN:HCOOH 0.1% aqueous solution = 45:55, column temperature = 25°C, flow rate = 1 mL/min, retention time of ²¹¹At-labeled compound 2g = 14.8 to 16.6 minutes (peak top: 15.6 minutes)

Radio-TLC analysis conditions: developing solvent = hexane/AcOEt = 8:1, Rf value of ²¹¹At-labeled compound 2g = 0.45

### (Example 12)

### Production of methyl (S)-3-(4-(astato-²¹¹At)phenyl)-2-(tert-butoxycarbonyl)amino)propanoate (2c)

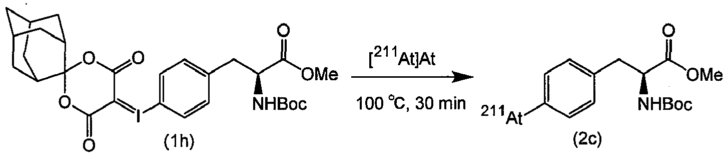

A reaction was carried out in the same manner as in Example 3, except that ²¹¹At/CHCl₃ solution (33 MBq) and an aryliodonium ylide 1h were used. The resulting reaction solution was analyzed by Radio-HPLC and Radio-TLC. A target ²¹¹At-labeled compound 2c was obtained with a Radio-TLC analysis radiochemical yield of 56%. The aryliodonium ylide 1h was produced referring to Non-patent Literatures 4 and 5.

### (Example 13)

### Production of ²¹¹At-labeled Compound (2a)

A reaction was carried out by the same method as described in Example 1, except that a ²¹¹At/CHCl₃ solution (39 MBq) was used in place of the ²¹¹At/CHCl₃ solution (43 MBq) and MeCN was used in place of DMF, and the resulting reaction solution was analyzed by Radio-HPLC and Radio-TLC. As a result, a target ²¹¹At-labeled compound 2a was obtained with a Radio-TLC analysis radiochemical yield of 82%.

### (Example 14)

### Production of ²¹¹At-labeled Compound (2a)

A reaction was carried out by the same method as described in Example 1, except that a ²¹¹At/CHCl₃ solution (27 MBq) was used in place of the ²¹¹At/CHCl₃ solution (43 MBq) and Et₄NHCO₃ was not used, and the resulting reaction solution was analyzed by Radio-HPLC and Radio-TLC. As a result, a target ²¹¹At-labeled compound 2a was obtained with a Radio-TLC analysis radiochemical yield of 23%.

### (Example 15)

### Production of ²¹¹At-labeled Compound (2a)

A reaction was carried out by the same method as described in Example 1, except that a ²¹¹At/CHCl₃ solution (52 MBq) was used in place of the ²¹¹At/CHCl₃ solution (43 MBq) and PPh₃ was not used, and the resulting reaction solution was analyzed by Radio-HPLC and Radio-TLC. As a result, a target ²¹¹At-labeled compound 2a was obtained with a Radio-TLC analysis radiochemical yield of 53%.

### (Example 16)

### Production of ²¹¹At-labeled Compound (2a)

A reaction was carried out by the same method as described in Example 1, except that a ²¹¹At/CHCl₃ solution (25 MBq) was used in place of the ²¹¹At/CHCl₃ solution (43 MBq) and the reaction temperature was changed from 100°C to 60°C, and the resulting reaction solution was analyzed by Radio-HPLC and Radio-TLC. As a result, a target ²¹¹At-labeled compound 2a was obtained with a Radio-TLC analysis radiochemical yield of 12%.

### (Example 17)

### Production of ²¹¹At-labeled Compound (2a)

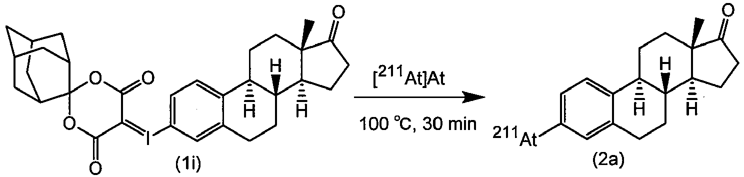

A reaction was carried out by the same method as described in Example 1, except that a ²¹¹At/CHCl₃ solution (40 MBq) was used in place of the ²¹¹At/CHCl₃ solution (43 MBq) and an aryliodonium ylide (1i) was used in place of the aryliodonium ylide (1a), and the resulting reaction solution was analyzed by Radio-HPLC and Radio-TLC. As a result, a target ²¹¹At-labeled compound 2a was obtained with a Radio-TLC analysis radiochemical yield of 12%. The aryliodonium ylide 1i was produced referring to Non-patent Literatures 4 and 5.

### (Example 18)

### Production of ²¹¹At-labeled Compound (2a)

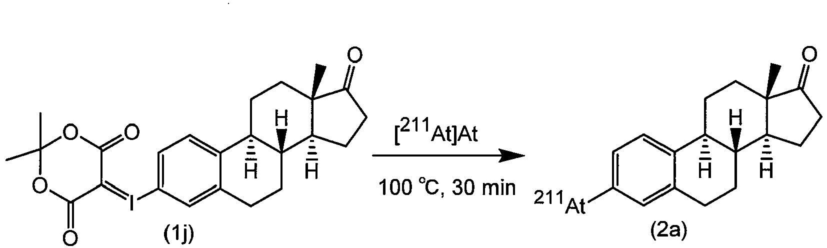

A reaction was carried out by the same method as described in Example 1, except that a ²¹¹At/CHCl₃ solution (32 MBq) was used in place of the ²¹¹At/CHCl₃ solution (43 MBq) and an aryliodonium ylide (1j) was used in place of the aryliodonium ylide (1a), and the resulting reaction solution was analyzed by Radio-HPLC and Radio-TLC. As a result, a target ²¹¹At-labeled compound 2a was obtained with a Radio-TLC analysis radiochemical yield of 12%. The aryliodonium ylide 1j was produced referring to Non-patent Literatures 4 and 5.

### Production of Aryliodonium Ylide (1k) Having Solid-Phase Support

An aryliodonium ylide (1k) having a solid-phase support can be produced by, for example, producing a Meldrum's acid derivative (12-8), subsequently allowing this Meldrum's acid derivative (12-8) to react with an azide (12-10) having the solid-phase support to produce a Meldrum's acid precursor (12) having the solid-phase support, and then allowing this Meldrum's acid precursor (12) having the solid-phase support to react with an aromatic precursor (11).

A synthesis scheme of the Meldrum's acid derivative (12-8) is shown below (Scheme 1).

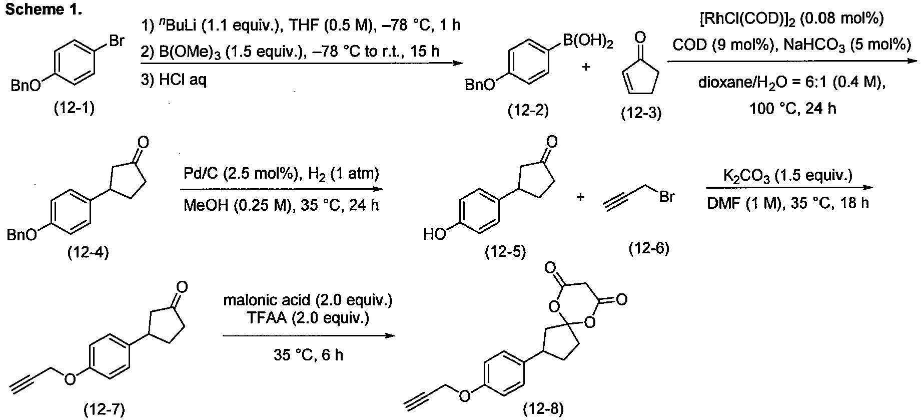

The steps of producing the Meldrum's acid derivative (12-8) will now each be described in detail.

### Production of (4-(benzyloxy)phenyl)boronic acid (12-2)

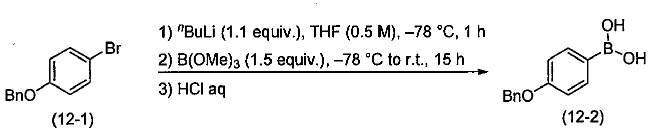

After dissolving 1-(benzyloxy)-4-bromobenzene (12-1) (25.1 g, 95.4 mmol, 1.0 equiv.) in THF (191 mL) and cooling the resultant to -78°C, *ⁿ*BuLi (2.67 M in hexane, 39,3 mL, 105 mmol) was added dropwise thereto. The resultant was stirred for 1 hour, and B(OMe)₃ (16 mL, 143 mmol) was subsequently added dropwise thereto, followed by 15-hour stirring at room temperature. The reaction solvent was removed by distillation under reduced pressure, and the resultant was diluted with ethyl acetate and a 10% aqueous hydrochloric acid solution (200 mL). A compound was extracted by a liquid separation operation using ethyl acetate, and the resulting organic layer was washed with water and a saturated aqueous sodium chloride solution and subsequently dried over sodium sulfate. The solvent was removed by distillation under reduced pressure, and the thus obtained solid was dissolved in diethyl ether (140 mL), followed by 15-minute stirring at room temperature. Hexane (240 mL) to the resultant, which was subsequently stirred for 15 minutes. The thus precipitated solid was recovered by vacuum filtration, whereby a target (4-(benzyloxy)phenyl)boronic acid (12-2) was obtained (18.1 g, 83%) .

### Production of 3-(4-(benzyloxy)phenyl)cyclopentan-1-one (12-4)

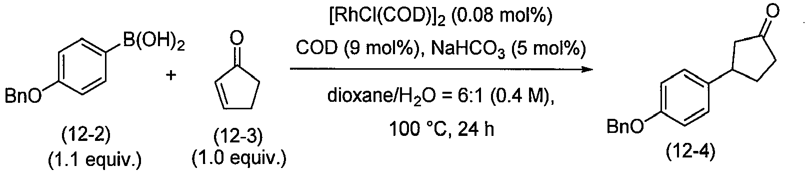

The thus obtained (4-(benzyloxy)phenyl)boronic acid (12-2) (18.1 g, 79.4 mmol), NaHCO₃ (303 mg, 3.6 mmol), [Rh(cod)Cl]₂ (28.1 mg, 0.057 mmol), and 1,5-cyclooctadiene (797 µL, 6.5 mmol) were added to a mixed solution of 1,4-dioxane and water (1,4-dioxane:water = 6:1, 180 mL). After stirring the resultant at room temperature for 5 minutes, cyclopent-2-en-1-one (12-3) (6.0 mL, 72.2 mmol, 1.0 equiv.) was added thereto, followed by 24-hour stirring at 100°C in an argon gas atmosphere. The resultant was cooled and then diluted with a mixed solution of water and a saturated aqueous sodium chloride solution, as well as ethyl acetate. A compound was extracted by a liquid separation operation using ethyl acetate, and the resulting organic layer was washed with water and a saturated aqueous sodium chloride solution and subsequently dried over sodium sulfate. The solvent was removed by distillation under reduced pressure, and the thus obtained solid was purified by a recrystallization operation (with heated ethyl acetate and hexane), whereby a target 3-(4-(benzyloxy)phenyl)cyclopentan-1-one (12-4) was obtained (16.0 g, 83%).

### Production of 3-(4-hydroxyphenyl)cyclopentan-1-one (12-5)

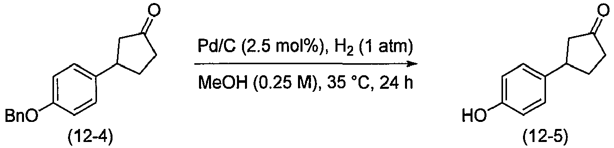

After suspending the thus obtained (3-(4-benzyloxy)phenyl)cyclopentan-1-one (12-4) (7.0 g, 26.3 mmol, 1.0 equiv.) in methanol (105 mL), Pd/C (10% Pd and 50% H₂O, 1.4 g, 0.66 mmol) was added, and the resultant was stirred at 35°C for 24 hours in a hydrogen gas atmosphere. After cooling the resultant, the resulting solid was recovered by celite filtration, and the solvent was removed by distillation under reduced pressure, whereby a target 3-(4-hydroxyphenyl)cyclopentan-1-one (12-5) was obtained (6.0 g, quant).

### Production of 3-(4-(prop-2-yn-1-yloxy)phenyl)cyclopentan-1-one (12-7)

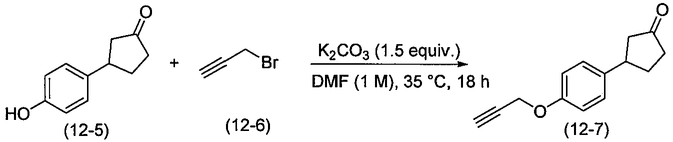

The thus obtained 3-(4-hydroxyphenyl)cyclopentan-1-one (12-5) (6.0 g, 26.3 mmol, 1.0 equiv.) and potassium carbonate (5.5 g, 39.5 mmol) were suspended in DMF (26.3 mL). The resultant was stirred at 35°C for 10 minutes, and 3-bromoprop-1-yne (12-6) (2.9 mL, 39.5 mmol) was subsequently added dropwise thereto. The resultant was stirred at 35°C for 18 hours and then diluted with water and ethyl acetate. A compound was extracted by a liquid separation operation using ethyl acetate, and the resulting organic layer was washed with water and a saturated aqueous sodium chloride solution and subsequently dried over sodium sulfate. The solvent was removed by distillation under reduced pressure, and the thus obtained residue was purified by silica gel column chromatography (hexane/ethyl acetate = 1/1), whereby a target 3-(4-(prop-2-yn-1-yloxy)phenyl)cyclopentan-1-one (12-7) was obtained (5.3 g, 93%) .

### Production of 2-(4-(prop-2-yn-1-yloxy)phenyl)-6,10-dioxaspiro[4.5]decane-7,9-dione (12-8)

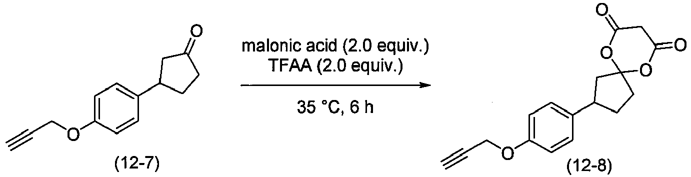

Malonic acid (2.1 g, 20 mmol) and trifluoroacetic anhydride (2.8 mL, 20 mmol) were mixed and stirred at 35°C for 10 minutes. After an addition of 3-(4-(prop-2-yn-1-yloxy)phenyl)cyclopentan-1-one (12-7) (2.1 g, 10 mmol, 1.0 equiv.) thereto, the resultant was stirred at 35°C for 6 hours. The resulting reaction solution was diluted with dichloromethane, washed with water and a saturated aqueous sodium chloride solution, and then dried over sodium sulfate. The solvent was removed by distillation under reduced pressure, and the thus obtained residue was purified by silica gel column chromatography (hexane/ethyl acetate = 1/1), whereby a target 2-(4-(prop-2-yn-1-yloxy)phenyl)-6,10-dioxaspiro[4.5]decane-7,9-dione (12-8) was obtained (1.85 g, 62%).

A synthesis scheme for producing the aryliodonium ylide (1k) having a solid-phase support (e.g., a polystyrene resin) from the above-obtained Meldrum's acid derivative (12-8) is shown below (Scheme 2).

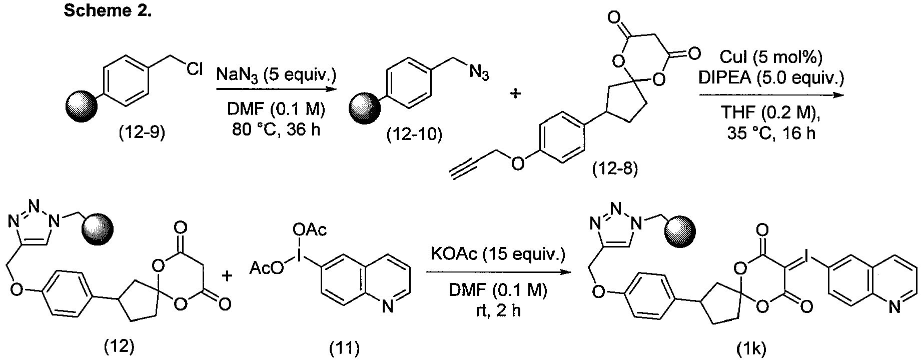

The steps will now each be described in detail.

### Production of Polystyrene Resin-Containing Azide (12-10)

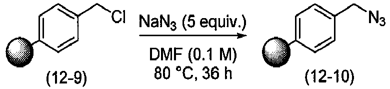

A chloromethyl polystyrene resin (12-9) (TCI, product code: C1643, 1.5 to 1.8 mmol/g, 278 mg) was swollen at room temperature for 1 hour with an addition of DMF (5 mL). To this resin, sodium azide (163 mg, 2.5 mmol) was added, and the resultant was stirred at 80°C for 36 hours. After cooling, the resin was washed with water (2 mL × 3), DMF (2 mL × 3), methanol (2 mL × 2), and diethyl ether (2 mL × 2). The resultant was dried at 40°C for 1 hour under reduced pressure to obtain a target polystyrene resin-containing azide (12-10) (283 mg).

### Production of Polystyrene Resin-Containing Meldrum's Acid Precursor (12)

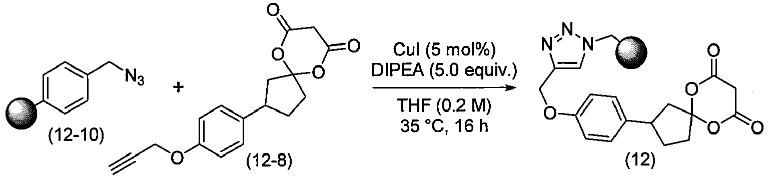

The polystyrene resin-containing azide (12-10) (283 mg) was swollen at room temperature for 1 hour with an addition of THF (2.5 mL). To this resin, copper iodide (4.8 mg, 0.025 mmol), N,N-diisopropylethylamine (435 µL, 2.5 mmol), and 2-(4-(prop-2-yn-1-yloxy)phenyl)-6,10-dioxaspiro[4.5]decane-7,9-dione (12-8) (300 mg, 1.0 mmol) were added, and the resultant was stirred at 35°C for 16 hours. After cooling, the resin was washed with DMF (2 mL × 3), dichloromethane (2 mL × 3), acetonitrile (2 mL × 3), methanol (2 mL × 2), and diethyl ether (2 mL × 2). The resultant was dried at 40°C for 1 hour under reduced pressure to obtain a target polystyrene resin-containing Meldrum's acid precursor (12) (493 mg).

### Production of Polystyrene Resin-Containing Aryliodonium Ylide (1k)

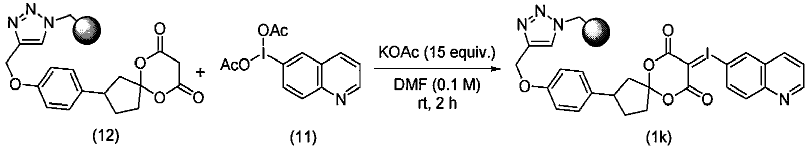

The polystyrene resin-containing Meldrum's acid precursor (12) (49.3 mg) was swollen at room temperature for 1 hour with an addition of DMF (0.5 mL). To this resin, potassium acetate (74 mg, 0.75 mmol) and quinolin-6-yl-13-iodanyl diacetate (aromatic precursor) (11) (37 mg, 0.1 mmol) were added, and the resultant was stirred at room temperature for 2 hours. The resin was washed with water (1 mL × 3), methanol (1 mL × 1), DMF (1 mL × 3), dichloromethane (1 mL × 3), and diethyl ether (1 mL × 2). The resultant was dried at room temperature for 1 hour under reduced pressure to obtain a target polystyrene resin-containing iodonium ylide (1k) (55.3 mg, 0.653 mmol/g).

### (Example 19)

### Production of ²¹¹At-labeled Compound (2e)

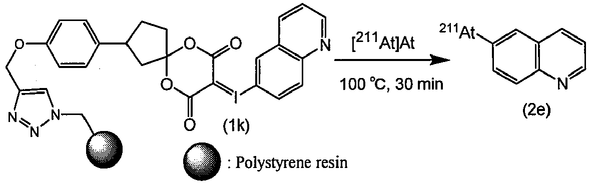

A reaction was carried out by the same method as described in Example 9, except that a ²¹¹At/CHCl₃ solution (61 MBq) was used in place of the ²¹¹At/CHCl₃ solution (43 MBq) and the polystyrene resin-containing (or polystyrene resin-supported) aryliodonium ylide (1k) was used in place of the aryliodonium ylide (le), and the resulting reaction solution was analyzed by Radio-HPLC and Radio-TLC. As a result, a target ²¹¹At-labeled compound 2e was obtained with a Radio-TLC analysis radiochemical yield of 10%.

In all of Examples 1 to 19, a reaction between an aromatic iodonium ylide optionally containing a heteroatom and [²¹¹At]astatine gave a corresponding aromatic astatine compound with an excellent yield. The aromatic iodonium ylides used in Examples 1 to 12 all had several functional groups (e.g., an ester group, an amino group, an imide group, a carbonyl group, and/or an oxo group); however, aromatic astatine compounds were produced with substantially no effect on these substituents.

### [Industrial Applicability]

The aromatic astatine compound production method according to one embodiment of the present invention includes allowing an aromatic iodonium ylide to react with [²¹¹At]astatine and, therefore, can utilize nucleophilic astatine that is a relatively safe and simple chemical species. In addition, not only it is not necessary to use a transition metal in a raw material of the reaction thereof, but also it is not necessary to use a transition metal as a catalyst. This enables to attain excellent safety and to simplify the resulting product, so that the product can be more easily purified. Further, more preferably, the reaction can achieve ²¹¹At labeling in a chemoselective and regioselective manner. Therefore, the aromatic astatine compound production method according to one embodiment of the present invention can be suitably employed for producing a ²¹¹At-labeled aromatic astatine compound.

### [Related Application]

This patent application claims priority under Article 4 of the Paris Convention or Article 41 of the Japan Patent Act based on Japanese Patent Application No. 2020-28536 filed in Japan on February 21, 2020, the entirety of which is incorporated herein by reference.

## Claims

1. A method of producing an aromatic astatine compound, the method comprising allowing an aromatic iodonium ylide to react with astatine to produce the aromatic astatine compound.

2. The method according to claim 1, wherein the aromatic iodonium ylide is represented by the following Formula (1): 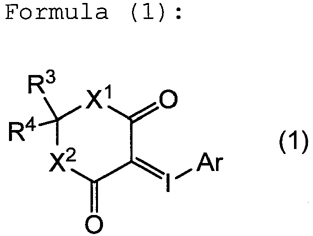 [wherein,
Ar represents an aromatic group optionally having a substituent and optionally having a heteroatom;
X¹ is selected from a group consisting of NR¹, O, and S;
X² is selected from a group consisting of NR², O, and S;
R¹ and R² are each independently selected from H, alkyls optionally having a substituent and optionally interrupted by a heteroatom, cycloalkyls optionally having a substituent and optionally interrupted by a heteroatom, and aromatic groups optionally having a substituent and optionally having a heteroatom; and
R³ and R⁴ are each independently selected from H, alkyls optionally having a substituent and optionally interrupted by a heteroatom, alkenyls optionally having a substituent and optionally interrupted by a heteroatom, alkynyls optionally having a substituent and optionally interrupted by a heteroatom, cycloalkyls optionally having a substituent and optionally interrupted by a heteroatom, and aromatic groups optionally having a substituent and optionally interrupted by a heteroatom, or
a combination of R³ and R⁴ is selected from oxo groups optionally having a substituent, wherein the oxo groups are formed by the combination of R³ and R⁴ together with the carbon atom to which R³ and R⁴ are bound, or
a combination of R³ and R⁴ is selected from cycloalkyls optionally having a substituent and optionally interrupted by a heteroatom, wherein the cycloalkyls are formed by the combination of R³ and R⁴ together with the carbon atom to which R³ and R⁴ are bound].

3. The method according to claim 2, wherein X¹ is O, and X² is O.

4. The method according to claim 2 or 3, wherein R³ and R⁴ are each independently selected from H, alkyls optionally having a substituent and optionally interrupted by a heteroatom, alkenyls optionally having a substituent and optionally interrupted by a heteroatom, alkynyls optionally having a substituent and optionally interrupted by a heteroatom, cycloalkyls optionally having a substituent and optionally interrupted by a heteroatom, and aromatic groups optionally having a substituent and optionally interrupted by a heteroatom.

5. The method according to claim 2 or 3, wherein the combination of R³ and R⁴ is selected from cycloalkyls optionally having a substituent and optionally interrupted by a heteroatom, wherein the cycloalkyls are formed by the combination of R³ and R⁴ together with the carbon atom to which R³ and R⁴ are bound.

6. The method according to claim 5, wherein the cycloalkyls optionally having a substituent and optionally interrupted by a heteroatom, wherein the cycloalkyls are formed by the combination of R³ and R⁴ together with the carbon atom to which R³ and R⁴ are bound, are selected from monocyclic, bicyclic, or tricyclic cycloalkyls.

7. The method according to any one of claims 2 to 6, wherein
the aromatic iodonium ylide comprises a solid-phase support, and
the solid-phase support is a part of a substituent of R¹, R², R³, R⁴, or the combination of R³ and R⁴ in Formula (1) .

8. The method according to claim 7, wherein the solid-phase support is a solid organic polymer compound.

9. The method according to claim 8, wherein the solid organic polymer compound is a polystyrene resin.

10. The method according to any one of claims 7 to 9, wherein
the aromatic iodonium ylide comprises a linker bound to the solid-phase support, and
the linker bound to the solid-phase support is a substituent of R¹, R², R³, R⁴, or the combination of R³ and R⁴ in Formula (1), is selected from a group consisting of an alkylene group, a cycloalkylene group, an alkenylene group, an arylene group, a heteroarylene group, a polymethylene group, a polyethylene glycol chain, and a combination thereof, and optionally has at least one of an ether group, an amino group, an amide group, an imide group, an ester group, and a combination thereof.

11. The method according to claim 10, wherein the linker is selected from a group consisting of an alkylene group, an arylene group, a heteroarylene group, and a combination thereof, and optionally has at least one ether group.

12. The method according to any one of claims 2 to 11, wherein the aromatic group (Ar) optionally having a substituent and optionally having a heteroatom is selected from an aryl group optionally having a substituent and a heteroaryl group optionally having a substituent.

13. The method according to claim 12, wherein the heteroaryl group optionally having a substituent is selected from sulfur-containing heteroaryl groups, oxygen-containing heteroaryl groups, nitrogen-containing heteroaryl groups, and heteroaryl groups containing two or more heteroatoms.

14. The method according to any one of claims 1 to 13, wherein the aromatic astatine compound is represented by the following Formula (2):
Formula (2): ²¹¹At-Ar
[wherein, Ar represents an aromatic group optionally having a substituent and optionally having a heteroatom].

15. The method according to any one of claims 1 to 14, comprising producing astatine using a cyclotron.
